# EUROPEAN PATENT SPECIFICATION

(11) **EP 4 304 679 B1**
(45) Date of publication and mention of the grant of the patent: **07.08.2024**
(21) Application number: 22718003.1
(22) Date of filing: 30.03.2022
(51) Int. Cl.: A61M 5/168

(54) **VISUAL COMMUNICATION OF INFUSION LINE CONDITIONS**
VISUELLE MITTEILIUNG DES ZUSTANDS DER INFUSIONSLEITUNG
COMMUNICATION VISUELLE DES CONDITIONS DE LA LIGNE DE PERFUSION

(30) Priority: 30.03.2021 US 202163168184 P
(43) Date of publication of application: 17.01.2024
(73) Proprietor: CareFusion 303, Inc., San Diego, CA 92130 (US)
(72) Inventor: MOORE, Ian, Limerick, Corbally 94 WY6T (IE)
(74) Representative: Zenz Patentanwälte Partnerschaft mbB
(86) International application number: PCT/US2022/022620
(87) International publication number: WO 2022/212560

(56) References cited:
- US-A1- 2013 208 497
- US-A1- 2018 177 938
- US-A1- 2019 217 006
- US-A1- 2021 052 807

## Description

### TECHNICAL FIELD

This application relates generally to associating and identifying intravenous (IV) administration lines.

### BACKGROUND

A patient receiving care in an intensive care units and/or an operating room may be connected to multiple IV administration lines. Ensuring that each administration line is correctly connected to the correct infusion fluid, and delivered to the patient at the correct infusion rate, may require substantial time and labor to track the path of each IV administration line between the patient and a respective infusion pump or fluid container. The presence of more than one IV administration line may lead to risks associated with misconnections or an IV administration line going to an infusion pump that is not programmed at the correct infusion rate for the infusion fluid. Tape or tags may be attached to the lines with written information on what medication container each line is attached. However, tape can fall off, be difficult or unintelligible to read, information recorded incorrectly. For safety reasons, IV administration lines are also monitored to ensure that the use of the IV administration lines does not extend beyond their intended usage period.

US 2018/177938 A1 discloses an IV line identification system for enabling ready identification of an IV line and its associated fluid source and output and for enabling distinguishing the IV line from other IV lines and their fluid sources and outputs. The IV line identification system includes a first light source and a second light source communicatively coupled to one another via a wireless connection. The light sources are configured such that when one is activated so as to generate a light signal, the other is automatically activated to generate a corresponding light signal. Each light source may be placed on opposite ends of an IV line to enable ready identification of each end of the same IV line.

US 2019/217006 A1 discloses fiber optically illuminated medical infusion tubes. Such an illuminated tube includes one or more infusion tubes, one or more optical fibers which is illuminated for the infusion tube and one or more light sources which lights up the optical fiber. The fiber is illuminated along the length of the infusion tube. The light sources have multiple light colors to identify each of the optical fiber. A light controller controls the lightings of the optical fiber, and the light controller can be programed for various lighting modes as desired.

US 2013/208497 A1 discloses an IV line identification system enabling ready identification of an IV line and its associated fluid source and output from other IV lines with their fluid sources and outputs. The IV line identification system includes lights that are coupled to a specific IV line. The lights can identify specific locations on the IV line, such as an entry end by an IV bag and an output end by a needle or port associated with a patient. The lights can also identify the portions of the IV line that enter and exit a pump. The lights can also be selectively turned on when a specific IV line needs to be identified, and turned off when the IV line does not need to be individually identified. The lights may be colored to distinguish between the lights associated with another IV line.

US 2021/052807 A1 discloses various embodiments for identification of medical fluid treatment to a medical patient. Systems and their operations are disclosed to identify a fluidic treatment protocol among a plurality of medical infusion lines. The system includes medical infusion pumps, illuminating medical infusion lines, and computing resources to initiate identification. Fluidic medical treatment is prone to error, cognitive load is elevated in healthcare operations, the various embodiments disclosed herein are systems and methods to decrease error and reduce cognitive load by identifying as a visual cue the medical fluid treatment protocol.

### SUMMARY

The invention is defined in the independent claims. Preferable embodiments are further laid out in the dependent claims. Labeling and reconciliation of IV lines (a process also known as "line tracing") may be done at least once per shift by a clinician. Line tracing is conducted to ensure that a particular medical fluid is connected to the intended channel for infusion to the intended patient. The clinician may check during line tracing that (i) the IV administration set has not been used beyond its intended usage period (or expired), (ii) the fluid in the fluid container has not expired, (iii) the IV administration line of the IV set is correctly labeled with stickers, (iv) the infusion pump is correctly labeled electronically, and (v) the IV site at the patient. The clinician may record such information as electronic documentation in the patient's electronic medical record (EMR).

Accordingly, there is a need for devices and methods that help to reduce the labor and increase efficiency associated with line reconciliation. More accurate line reconciliations also prevent accidental line swaps in which the wrong medical fluid is used in the infusion. Accidental line swaps may also cause the correct medical fluid to be infused at the wrong infusion rate through the wrong infusion pump to the patient. Such devices and methods may also help to improve the accuracy of expiry information associated with the IV set and/or the infusion medication to be infused that is provided to the clinician.

The disclosed subject matter relates to an infusion set comprising a fluid-carrying tubing, a light array coupled to the fluid-carrying tubing at a plurality of fastening points along a length of the fluid-carrying tubing, a pump interface and a controller, wherein the controller is configured to: receive a pumping parameter from the pump interface, the pumping parameter being generated by an infusion pump providing a fluid to a patient through the fluid-carrying tubing and representative of a property of the fluid that is measured by a sensor of the infusion pump, cause, responsive to receiving the pumping parameter, the light array to display, along the length of the fluid-carrying tubing, a light pattern representative of the pumping parameter. Other aspects include corresponding methods, apparatus, and computer program products for implementation of the corresponding infusion set and its features.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

For a better understanding of the various described implementations, reference should be made to the Description below, in conjunction with the following drawings. Like reference numerals refer to corresponding parts throughout the figures and description.
FIG. 1 depicts a medical administration system having four medical administration devices such as infusion pumps, according to aspects of the subject technology.
FIG. 2 depicts an example intravenous (IV) pole with multiple infusion devices (including pumps) hanging therefrom.
FIG. 3 depicts an example of an institutional patient care system of a healthcare organization, according to aspects of the subject technology.
FIGS. 4A and 4B depict an infusion line lighting system, according to various aspects of the subject technology.
FIG. 5 depicts an example attachment of a light array to a fluid infusion line, according to various aspects of the subject technology.
FIGS. 6A to 6D depict example light patterns produced by the disclosed controller and light array, according to various implementations of the subject technology.
FIG. 7 depicts an example process for optical communication of infusion line conditions, according to aspects of the subject technology.
FIG. 8 is a conceptual diagram illustrating an example electronic system for controlling a light array to optically communicate infusion line conditions, according to aspects of the subject technology.

### DESCRIPTION

Reference will now be made to implementations, examples of which are illustrated in the accompanying drawings. In the following description, numerous specific details are set forth in order to provide an understanding of the various described implementations. However, it will be apparent to one of ordinary skill in the art that the various described implementations may be practiced without these specific details. In other instances, well-known methods, procedures, components, circuits, and networks have not been described in detail so as not to unnecessarily obscure aspects of the implementations.

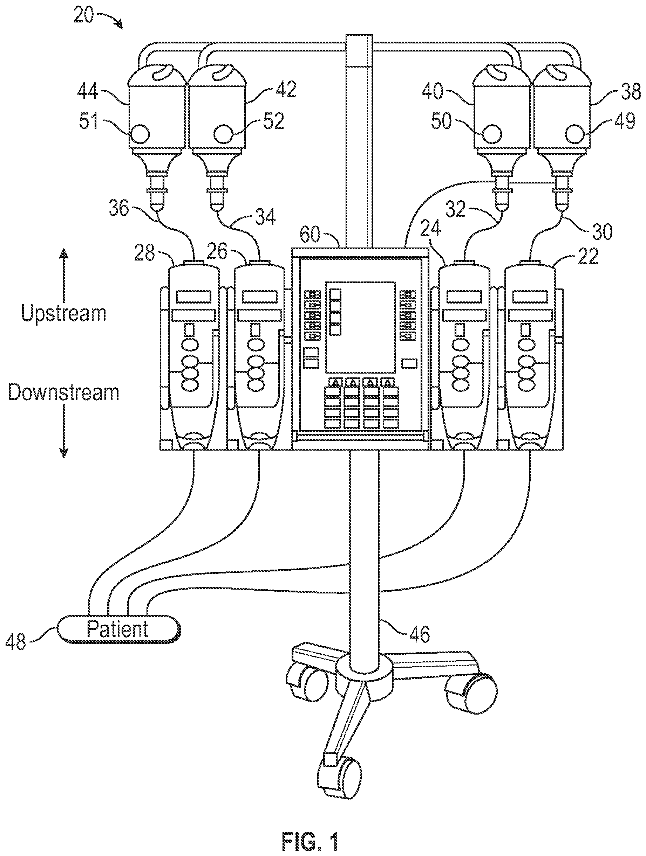

FIG. 1 depicts a medical administration system 20 having four medical administration devices 22, 24, 26, and 28, such as infusion pumps, each of which is in operative engagement with a respective fluid administration set 30, 32, 34, and 36. Medical fluid supplies or containers 38, 40, 42, and 44, which may take various forms but in this case are shown as inverted bottles, are suspended above the pumps. Fluid supplies may also take the form of bags or other types of containers. Both the infusion pumps 22, 24, 26 and 28 and the fluid containers 38, 40, 42, and 44 are mounted to a roller stand or IV pole 46.

Each administration set 30, 32, 34, and 36 is connected between a respective fluid container 38, 40, 42, and 44 and the same patient 48, so that the patient is connected to multiple fluid administration sets and receives fluids from multiple fluid containers (e.g., four in this example). A separate infusion pump 22, 24, 26, and 28 is used to infuse each of the fluids of the fluid containers into the patient. The infusion pumps are flow control devices that will act on the respective tube or fluid conduit of the fluid administration set to move the fluid from the fluid container through the conduit to the patient. Because individual pumps are used, each can be individually set to the pumping or operating parameters required for infusing the particular medical fluid from the respective fluid container into the patient at the particular rate prescribed for that fluid by the physician. Such medical fluids may include medications, drugs, nutrients, or other therapeutic fluids as previously mentioned.

Typically, medical fluid administration sets have more parts than those shown in FIG. 1A. Many have check valves, drip chambers, valved ports, connectors, and other devices well known to those skilled in the art. Sometimes, during the course of an infusion therapy regimen, a second drug is prescribed for infusion at the same time a first drug is being infused into a patient. Attention is directed to U.S. Pat. No. 9,307, 907 entitled "System and method for dynamically adjusting patient therapy" to Condurso et al., incorporated herein by reference, in which further details about how secondary infusions may be accomplished using a "Y-Site" connector that provides access to the primary infusion source line are provided. These other devices have not been included in the drawings so as to preserve clarity of illustration. However, those skilled in the art will understand that the inclusion of such other devices may often occur.

In accordance with the background discussed above, it is desirable to verify that each fluid container 38, 40, 42 and 44 is associated with the correct patient 48, that the pumping parameters for a given medical fluid have been correctly programmed into the infusion pump 22, 24, 26 or 28, and that each fluid container is correctly connected to the appropriately programmed pump. As will be discussed in more detail below, the present invention allows an infusion fluid line to be more easily and accurately associated with an infusion device which controls its fluid flow and/or with the fluid container from which the fluid originates. Data devices associated with the medical fluid containers, such as acoustic transducers 49, 50 51, and 52 shown mounted on the bottles, associated with the patient 48, may communicate relevant administration data to the pumps for verifying that the medical administration system 20 has been connected correctly. Relevant administration data may include various data related to the administration of medical fluid to a particular patient. For example, relevant administration data may include drug identification, patient identification, and other information believed to be relevant.

It should be noted that the drawing of FIG. 1A is not to scale and that distances have been compressed and sizes have been exaggerated for the purpose of clarity. In an actual setting, the distance between the containers 38, 40, 42, and 44 and the infusion pump 22, 24, 26, and 28 could be much greater. There would be more of an opportunity for the tubes of the administration sets 30, 32, 34, and 36 to become intertwined with each other when all four are dangling from the containers, which can cause confusion as to which tube should be in which infusion pump. The opportunity for confusion increases as the number of tubes increases. However, it should also be understood that the present invention is also useful in cases where a single pump and single medical fluid container are involved, as the system may also be used to confirm that the correct medication has arrived for the patient and that the infusion parameters have been correctly programmed into the pump.

The terms "upstream" and "downstream" as shown in FIG. 1A and as used herein in various places is meant to provide an indication of relative positioning as well as indicate the positions of certain specific devices. For example, the patient is located "downstream" from the pump and is also "downstream" from the container. The pump is located "upstream" from the patient, as is the container. On the other hand, there is an "upstream data reader device" and a "downstream data reader device"" which denote their relative positions on the pump.

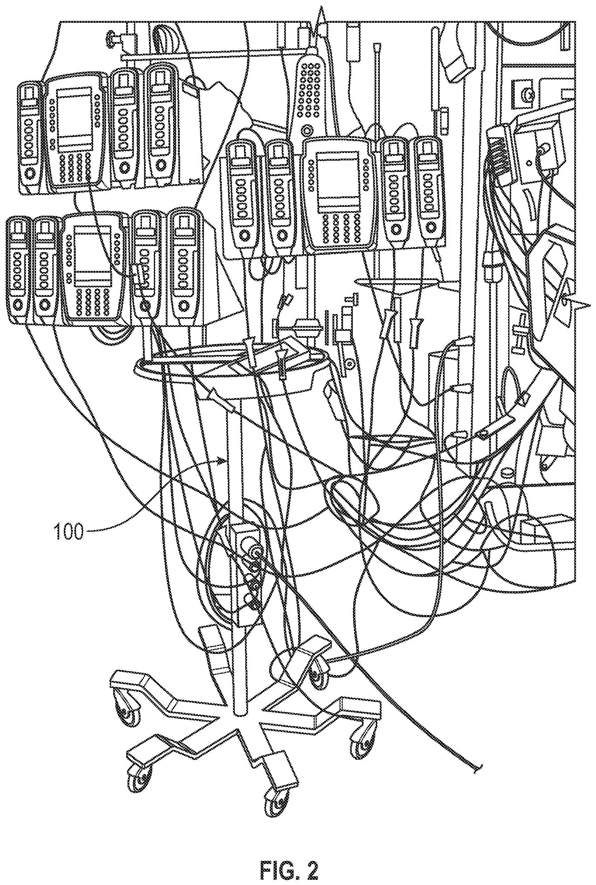

FIG. 2 depicts an example intravenous (IV) pole 100 with multiple infusion devices (including pumps) hanging therefrom. Each infusion device may include multiple pumps and thus multiple infusion lines. In an intensive care unit (ICU) or emergency room environment multiple infusion devices may be hung or attached to a single IV pole. When multiple infusion lines are connected to each infusion device (e.g. by way of multiple infusion modules) the infusion lines may become entangled with each other and it becomes more difficult to determine which line is connected to which device (or module). This scenario is commonly known as "infusion line spaghetti".

Individual clasp type organizers which may organize a limited amount of infusion lines (e.g. four) at a single point in the lines are difficult to implement in an emergency environment and do not prevent entanglement or twisting of the infusion lines between each organizer. Such organizers also do not bridge the gap between the IV pole and the patient bed, and thus do not address the "spaghetti" of lines from the IV pole to the patient.

The subject technology identifies infusion lines despite any clutter, thereby minimizing potential risks involved with identifying misplaced or defective infusion lines. The subject technology includes an infusion light array that continuously illuminates a single IV line from a pump on an IV pole to the patient or patient bed.

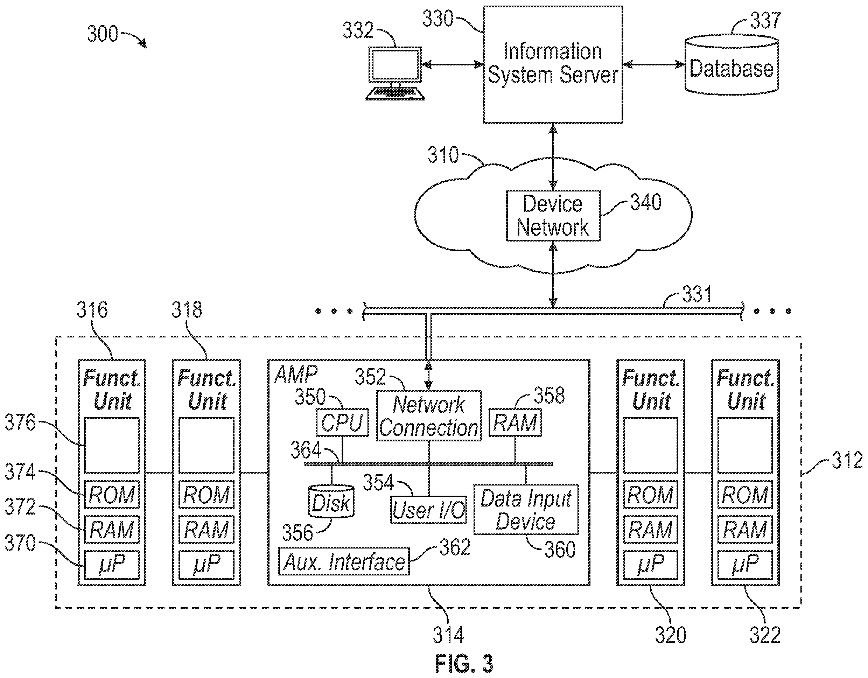

FIG. 3 depicts an example of an institutional patient care system 300 of a healthcare organization, according to aspects of the subject technology. In FIG. 3, a patient care device (or "medical device" generally) 312 is connected to a hospital network 10. The term patient care device (or "PCD") may be used interchangeably with the term patient care unit (or "PCU"), either which may include various ancillary medical devices such as an infusion pump, a vital signs monitor, a medication dispensing device (e.g., cabinet, tote), a medication preparation device, an automated dispensing device, a module coupled with one of the aforementioned (e.g., a syringe pump module configured to attach to an infusion pump), or other similar devices. Each element 312 is connected to an internal healthcare network 310 by a transmission channel 331. Transmission channel 331 is any wired or wireless transmission channel, for example an 802.11 wireless local area network (LAN). In some implementations, network 310 also includes computer systems located in various departments throughout a hospital. For example, network 310 of FIG. 3 optionally includes computer systems associated with an admissions department, a billing department, a biomedical engineering department, a clinical laboratory, a central supply department, one or more unit station computers and/or a medical decision support system. As described further below, network 310 may include discrete subnetworks. In the depicted example, network 310 includes a device network 340 by which patient care devices 312 (and other devices) communicate in accordance with normal operations.

Additionally, institutional patient care system 100 may incorporate a separate information system server 330, the function of which will be described in more detail below. Moreover, although the information system server 330 is shown as a separate server, the functions and programming of the information system server 330 may be incorporated into another computer, if such is desired by engineers designing the institution's information system. Institutional patient care system 300 may further include one or multiple device terminals 332 for connecting and communicating with information system server 330. Device terminals 332 may include personal computers, personal data assistances, mobile devices such as laptops, tablet computers, augmented reality devices, or smartphones, configured with software for communications with information system server 330 via network 310.

Patient care device 312 includes a system for providing patient care, such as that described in Eggers et al., which is incorporated herein by reference for that purpose. Patient care device 312 may include or incorporate pumps, physiological monitors (e.g., heart rate, blood pressure, ECG, EEG, pulse oximeter, and other patient monitors), therapy devices, and other drug delivery devices may be utilized according to the teachings set forth herein. In the depicted example, patient care device 312 comprises a control module 314, also referred to as interface unit 314, connected to one or more functional modules 316, 318, 320, 322. Interface unit 314 includes a central processing unit (CPU) 350 connected to a memory, for example, random access memory (RAM) 358, and one or more interface devices such as user interface device 354, a coded data input device 360, a network connection 352, and an auxiliary interface 362 for communicating with additional modules or devices. Interface unit 314 also, although not necessarily, includes a main non-volatile storage unit 356, such as a hard disk drive or non-volatile flash memory, for storing software and data and one or more internal buses 364 for interconnecting the aforementioned elements.

In various implementations, user interface device 354 is a touch screen for displaying information to a user and allowing a user to input information by touching defined areas of the screen. Additionally or in the alternative, user interface device 354 could include any means for displaying and inputting information, such as a monitor, a printer, a keyboard, softkeys, a mouse, a track ball and/or a light pen. Data input device 360 may be a bar code reader capable of scanning and interpreting data printed in bar coded format. Additionally or in the alternative, data input device 360 can be any device for entering coded data into a computer, such as a device(s) for reading a magnetic strips, radio-frequency identification (RFID) devices whereby digital data encoded in RFID tags or smart labels (defined below) are captured by the reader 360 via radio waves, PCMCIA smart cards, radio frequency cards, memory sticks, CDs, DVDs, or any other analog or digital storage media. Other examples of data input device 360 include a voice activation or recognition device or a portable personal data assistant (PDA). Depending upon the types of interface devices used, user interface device 354 and data input device 360 may be the same device. Although data input device 360 is shown in FIG. 3 to be disposed within interface unit 314, it is recognized that data input device 360 may be integral within a pharmacy system or located externally and communicating with pharmacy system through an RS-232 serial interface or any other appropriate communication means. Auxiliary interface 362 may be an RS-232 communications interface, however any other means for communicating with a peripheral device such as a printer, patient monitor, infusion pump or other medical device may be used without departing from the subject technology. Additionally, data input device 360 may be a separate functional module, such as modules 316, 318, 320 and 322, and configured to communicate with controller 314, or any other system on the network, using suitable programming and communication protocols.

Network connection 352 may be a wired or wireless connection, such as by Ethernet, WiFi, BLUETOOTH, an integrated services digital network (ISDN) connection, a digital subscriber line (DSL) modem or a cable modem. Any direct or indirect network connection may be used, including, but not limited to a telephone modem, an MIB system, an RS232 interface, an auxiliary interface, an optical link, an infrared link, a radio frequency link, a microwave link or a WLANS connection or other wireless connection.

Functional modules 316, 318, 320, 322 are any devices for providing care to a patient or for monitoring patient condition. As shown in FIG. 3, at least one of functional modules 316, 318, 320, 322 may be an infusion pump module such as an intravenous infusion pump for delivering medication or other fluid to a patient. For the purposes of this discussion, functional module 316 is an infusion pump module. Each of functional modules 318, 320, 322 may be any patient treatment or monitoring device including, but not limited to, an infusion pump, a syringe pump, a PCA pump, an epidural pump, an enteral pump, a blood pressure monitor, a pulse oximeter, an EKG monitor, an EEG monitor, a heart rate monitor or an intracranial pressure monitor or the like. Functional module 318, 320 and/or 322 may be a printer, scanner, bar code reader or any other peripheral input, output or input/output device.

Each functional module 316, 318, 320, 322 communicates directly or indirectly with interface unit 314, with interface unit 314 providing overall monitoring and control of device 312. Functional modules 316, 318, 320, 322 may be connected physically and electronically in serial fashion to one or both ends of interface unit 314 as shown in FIG. 3, or as detailed in Eggers et al. However, it is recognized that there are other means for connecting functional modules with the interface unit that may be utilized without departing from the subject technology. It will also be appreciated that devices such as pumps or patient monitoring devices that provide sufficient programmability and connectivity may be capable of operating as stand-alone devices and may communicate directly with the network without connected through a separate interface unit or control unit 314. As described above, additional medical devices or peripheral devices may be connected to patient care device 312 through one or more auxiliary interfaces 362.

Each functional module 316, 318, 320, 322 may include module-specific components 376, a microprocessor 370, a volatile memory 372 and a nonvolatile memory 374 for storing information. It should be noted that while four functional modules are shown in FIG. 3, any number of devices may be connected directly or indirectly to central controller 314. The number and type of functional modules described herein are intended to be illustrative, and in no way limit the scope of the subject technology. Module-specific components 376 include any components necessary for operation of a particular module, such as a pumping mechanism for infusion pump module 316.

While each functional module may be capable of a least some level of independent operation, interface unit 314 monitors and controls overall operation of device 312. For example, as will be described in more detail below, interface unit 314 provides programming instructions to the functional modules 316, 318, 320, 322 and monitors the status of each module.

Patient care device 312 is capable of operating in several different modes, or personalities, with each personality defined by a configuration database. The configuration database may be a database 356 internal to patient care device, or an external database 337. A particular configuration database is selected based, at least in part, by patient-specific information such as patient location, age, physical characteristics, or medical characteristics. Medical characteristics include, but are not limited to, patient diagnosis, treatment prescription, medical history, medical records, patient care provider identification, physiological characteristics or psychological characteristics. As used herein, patient-specific information also includes care provider information (e.g., physician identification) or a patient care device's 312 location in the hospital or hospital computer network. Patient care information may be entered through interface device 354, or the data input device 360, or auxiliary interface 362, and may originate from anywhere in network 310, such as, for example, from a pharmacy server, admissions server, laboratory server, and the like.

Medical devices incorporating aspects of the subject technology may be equipped with a network interface module (NIM), allowing the medical device to participate as a node in a network. While for purposes of clarity the subject technology will be described as operating in an Ethernet network environment using the Internet Protocol (IP), it is understood that concepts of the subject technology are equally applicable in other network environments, and such environments are intended to be within the scope of the subject technology.

Data to and from the various data sources can be converted into network-compatible data with existing technology, and movement of the information between the medical device and network can be accomplished by a variety of means. For example, patient care device 312 and network 310 may communicate via automated interaction, manual interaction or a combination of both automated and manual interaction. Automated interaction may be continuous or intermittent and may occur through direct network connection 352 (as shown in FIG. 3), or through RS232 links, MIB systems, RF links such as BLUETOOTH, IR links, WLANS, digital cable systems, telephone modems or other wired or wireless communication means. Manual interaction between patient care device 312 and network 310 involves physically transferring, intermittently or periodically, data between systems using, for example, user interface device 354, coded data input device 360, bar codes, computer disks, portable data assistants, memory cards, or any other media for storing data. The communication means in various aspects is bidirectional with access to data from as many points of the distributed data sources as possible. Decision-making can occur at a variety of places within network 310. For example, and not by way of limitation, decisions can be made in a remote data server, a hospital department or unit stations, or within patient care device 312 itself.

All direct communications with medical devices operating on a network in accordance with the subject technology may be performed through information system server 330, known as the remote data server (RDS). In accordance with aspects of the subject technology, network interface modules incorporated into medical devices such as, for example, infusion pumps or vital signs measurement devices, ignore all network traffic that does not originate from an authenticated RDS. The primary responsibilities of the RDS of the subject technology are to track the location and status of all networked medical devices that have NIMs, and maintain open communication.

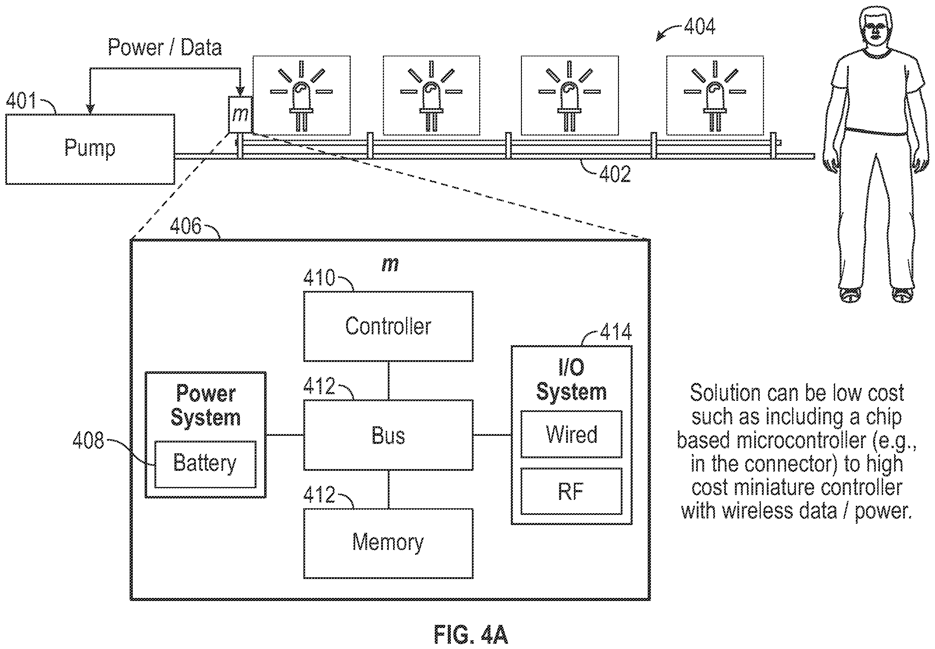

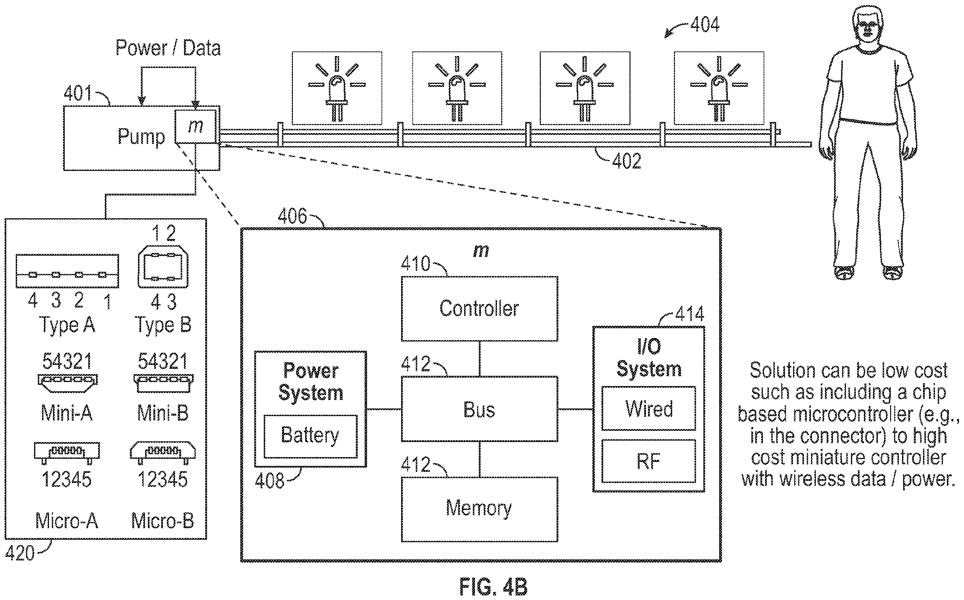

FIGS. 4A and 4B depict an infusion line lighting system 400, according to various aspects of the subject technology. FIG. 4A depicts a self-contained lighting system 400 that is external to the components of an infusion device or hospital system such as information server 330 over device network 340. As will be described further, system 400 communicates with a pump 401 to determine various pump conditions related to an infusion line 402 and visually identifies the pump conditions to a user via a light array 404 attached to infusion line 402.

System 400 may be connected to pump 401 or networked hospital control system 330. System 400 includes a fluid-carrying tubing 402 with light array 404 coupled to the fluid-carrying tubing along a length of the fluid-carrying tubing 402, and a line set controller 406. Line set controller 406 may include a power system 408, a microprocessor-based controller 410, a (non-transitory machine readable) memory device 412, an input/output (I/O) interface 414, and a bus 416 for communications between the same. System 400 may connect to pump 401 via I/O interface 414. In this regard, I/O interface 414 may include a USB type connector that plugs into a corresponding receiving connector in pump 401 (see FIG. 4B). The I/O interface 414 may provide input and/or output of data to the system 400. The I/O interface 414 may additionally or alternatively provide input and/or output of power to the system 400. Additionally or in the alternative, I/O interface may include a wireless network interface that wirelessly connects to pump 401 or hospital information system 330 to receive pump information and line set conditions.

According to various aspects, microprocessor-based controller 410 is configured to receive a pumping parameter from input/output (I/O) interface 414. The pumping parameter may be generated by an infusion pump 401, which may be currently providing a fluid to a patient through fluid-carrying tubing 402. The pumping parameter may be an operational parameter such as a flow rate or bolus amount, or may indicate a type of fluid (e.g. medication) being administered through tubing 402. According to various aspects, the pumping parameter may be representative of a fault condition in an IV line set. For example, if the pump detects an occlusion or that there is air in the line then the pumping parameter may be set to a value representative of the occlusion condition or an air-in-line condition. The pumping parameter may be indicative of the fluid running low beyond a predetermined amount or the pump 401 signaling that the fluid should be replenished. The pumping parameter may indicate the IV line set needs to be changed. According to some implementations pumping parameter may be an illumination command to identify the corresponding line set 402 attached to light array 404 among many line sets.

Pump 401 may be configured to represent the foregoing conditions using predetermined values (e.g. in its communication protocol), each condition corresponding to a unique value. The values may also be stored in memory device 412 and, when received from pump 418 via I/O system 414, controller 410 may look up the received value, and index the received value to a predetermined action. According to various implementations, each action corresponds to a predetermined light pattern to be displayed on light array 404. Line set controller 410 is thereby configured to encode the received pumping parameter into a lighting pattern and display the lighting pattern using light array 404. In this manner, the lights of light array 404 may be modulated to flash or blink, light up and cascade in a certain direction, function as a static rope light, change colors, pulse at a predetermined frequency, etc.

Line set controller 406 (including microprocessor-based controller 410) communicates with light array 404 via bus 416 and I/O interface 414. I/O interface 414 may include a connector for connecting to an electrical bus of light array 404 which is used to control the individual lights in light array 404. Responsive to receiving the pumping parameter, controller 410 causes the light array to display, along the length of the fluid-carrying tubing, a light pattern representative of the pumping parameter. For example, a subset of lights in light array 404 may be modulated to display a sequential cascade of lights that travels down a portion of infusion line or the length of the infusion line (in some implementations the pattern may be similar to a snake traveling down the infusion line). In one example where the pumping parameter to be displayed is an infusion rate, the pattern may repeat or be modulated to cascade at a rate according to the detected infusion rate.

In some implementations, pump 418 or controller 410 may be associated with a sensor configured to measure a property of the fluid in the fluid-carrying tubing 402. For example, the sensor may be a flow sensor configured to (e.g. optically) detect a flow of the fluid. The sensor may measure the property of the fluid and communicate the measured property to pump 418 or controller 410. The measured property may then be provided to controller 410 in the pumping parameter.

In some implementations, controller 410 may be configured to store a history of pumping parameters in memory device 412, including the currently received pumping parameters. In this regard, controller 410 may review the stored parameters, identify a predetermined pattern in the stored parameters representative of a predetermined action, and generate the light pattern according to the identified pattern. For example, if there is a fault condition, controller 410 may cause light array 404 to flash thereby indicating which infusion line 402 within a spaghetti of infusion lines is the line that needs attention.

FIG. 4B depicts a self-contained lighting system 400 that is integrated with the components of an infusion pump 401 or hospital system such as information server 330 over device network 340. As shown, pump 401 may include one or more various types of device connections 420 into which light array may be connected for control by controller 410. With reference back to FIG. 4A, connector 420 may also be used to connect pump 401 to line set controller 406.

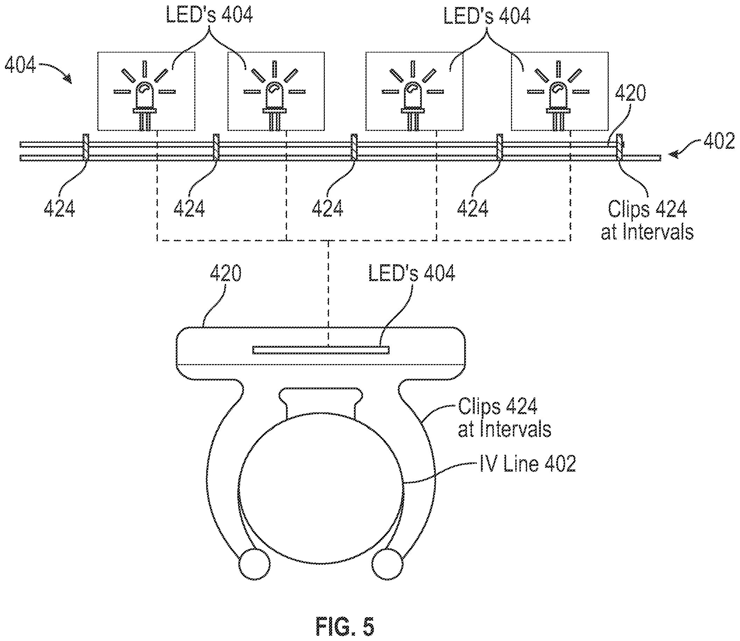

FIG. 5 depicts an example attachment of a light array 404 to a fluid infusion line 402, according to various aspects of the subject technology. According to various examples, light array 404 includes a multitude of lights embedded in a light string. The lights may include light emitting diodes (LEDs), including surface mounted diode (SMD) LEDs, or may include curved filaments lined with LED diodes. Each LED may incorporate several emitters of different colors to form a multi-colored LED. The LEDs may be formed by nano structures such as a quantum dot that emits light (e.g. responsive to gathering a positive charge).

The LEDs may be embedded in a flexible translucent 422 casing that runs along the length of tubing 402. In the depicted example, casing 422 is secured to line 402 by fastening points 424, which may include clips integrated with casing 422. Each clip 424 may (e.g. loosely but securely) clip onto the fluid-carrying tubing at periodic intervals along the fluid-carrying tubing in a manner not to constrict tubing 402 or otherwise impeded the fluid traveling therein. In some implementations, a backing of casing 422 may be opaque to protect light sensitive medications administered through line 402. In some implementations, casing 422 may be a sheath that slides over tubing 402. In some implementations, light array 402 includes the lights being embedded within the fluid-carrying tubing 402 itself.

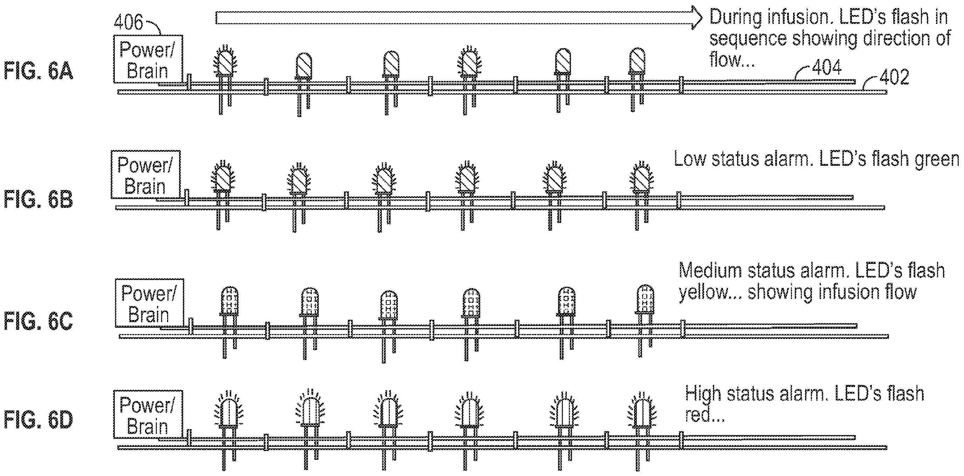

FIGS. 6A to 6D depict example light patterns produced by the disclosed controller and light array, according to various implementations of the subject technology. FIG. 6A depicts controller 406 causing the lights (e.g. LEDs) of light array to flash in sequence showing a direction of the flow of fluid within tubing 402. In this example, the lights may "follow" the direction of fluid flow, and may speed up or slow down according to the flow rate.

FIG. 6B depicts a low status alarm in which all or a subset of lights may flash in a first, non-alarming color such as green. This pattern may be displayed, for example, when controller 406 is caused to identify an infusion line. For example, pumping parameter may be indicative of a request to identify an infusion line. This may be by way of a predetermined input into a control panel 354 of pump 401, or by pump 401 or controller 406 receiving a wireless signal from a remote device (e.g. a mobile device or system 330) to identify the line of the pump.

FIG. 6C depicts a medium status alarm in which all or a subset of the lights flash in a second color such as yellow. This pattern may be displayed, for example, when controller 406 is caused to identify the infusion flow, or a minor anomaly in the infusion flow. FIG. 6D depicts a high status alarm in which all or a subset of the lights flash in a third color such as red. This pattern may be displayed, for example, when controller 406 is caused to identify an occlusion, air-in-line, or other fault condition. According to some implementations, pump 401 and/or controller 406 may be configured to identify the location of the occlusion by way of the light pattern. For example, a subset of lights between the pump and fluid source or container may be modulated to indicate the cause of the occlusion or fault condition is upstream from the pump, and a subset of lights between the pump and the patient may be modulated to indicate that the cause of the occlusion or fault condition is located downstream of the pump. The lights may further be modulated in sequence to show a further directional positioning of the fault condition for more accurate and immediate location by the clinician. A few lights (e.g. one, three, four, or five) may be modulated or an entire segment.

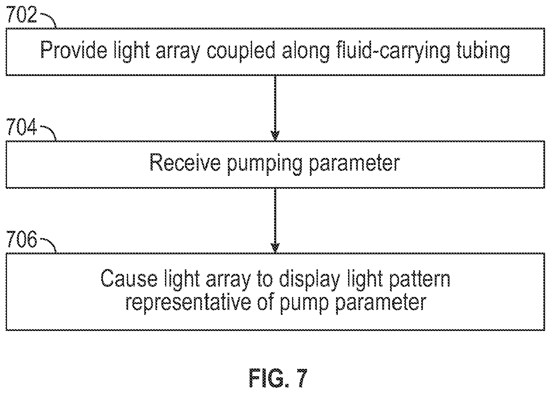

FIG. 7 depicts an example process 700 for optical communication of infusion line conditions, according to aspects of the subject technology. For explanatory purposes, the various blocks of example process 800 are described herein with reference to FIGS. 1 to 6, and the components and/or processes described herein. The one or more of the blocks of process 800 may be implemented, for example, by one or more computing devices including, for example, medical device 12. In some implementations, one or more of the blocks may be implemented based on one or more machine learning algorithms. In some implementations, one or more of the blocks may be implemented apart from other blocks, and by one or more different processors or devices. Further for explanatory purposes, the blocks of example process 800 are described as occurring in serial, or linearly. However, multiple blocks of example process 800 may occur in parallel. In addition, the blocks of example process 800 need not be performed in the order shown and/or one or more of the blocks of example process 800 need not be performed.

In the depicted example, a light array 404, configured to couple to a fluid-carrying tubing at a plurality of fastening points along a length of the fluid-carrying tubing 402, is provided (702). A controller 406 operably coupled to the light array receives a pumping parameter from a pump interface (704). The pumping parameter may be generated by an infusion pump 401 providing a fluid to a patient through the fluid-carrying tubing 402. Controller 406 causes, responsive to receiving the pumping parameter, the light array to display, along the length of the fluid-carrying tubing 402, a light pattern representative of the pumping parameter (706).

Many of the above-described devices, systems and methods, may also be implemented as software processes that are specified as a set of instructions recorded on a computer readable storage medium (also referred to as computer readable medium), and may be executed automatically (e.g., without user intervention). When these instructions are executed by one or more processing unit(s) (e.g., one or more processors, cores of processors, or other processing units), they cause the processing unit(s) to perform the actions indicated in the instructions. Examples of computer readable media include, but are not limited to, CD-ROMs, flash drives, RAM chips, hard drives, EPROMs, etc. The computer readable media does not include carrier waves and electronic signals passing wirelessly or over wired connections.

The term "software" is meant to include, where appropriate, firmware residing in read-only memory or applications stored in magnetic storage, which can be read into memory for processing by a processor. Also, in some implementations, multiple software aspects of the subject disclosure can be implemented as sub-parts of a larger program while remaining distinct software aspects of the subject disclosure. In some implementations, multiple software aspects can also be implemented as separate programs. Finally, any combination of separate programs that together implement a software aspect described here is within the scope of the subject disclosure. In some implementations, the software programs, when installed to operate on one or more electronic systems, define one or more specific machine implementations that execute and perform the operations of the software programs.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

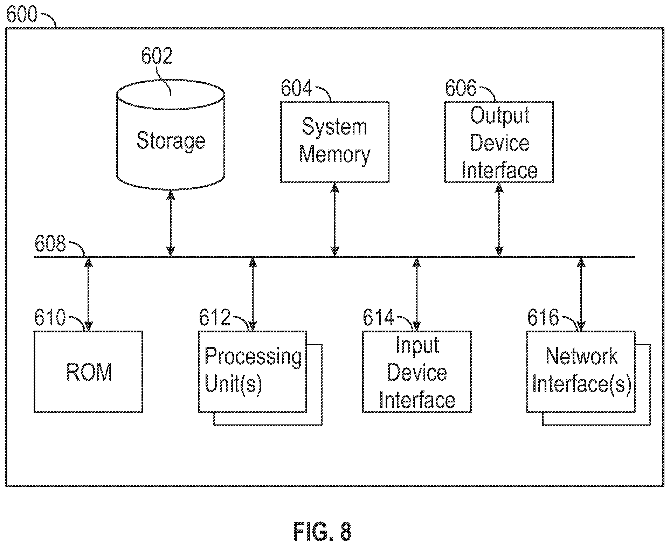

FIG. 8 is a conceptual diagram illustrating an example electronic system 600 for controlling a light array to optically communicate infusion line conditions, according to aspects of the subject technology. Electronic system 600 may be a computing device for execution of software associated with one or more components and processes provided by FIGS. 1 to 7, including but not limited to controller 406, or computing hardware within pump 401 or system 330. Electronic system 600 may be representative of a device used in connection or combination with the disclosure regarding FIGS. 1 to 7. In this regard, electronic system 600 may be a personal computer or a mobile device such as a smartphone, tablet computer, laptop, PDA, an augmented reality device, a wearable such as a watch or band or glasses, or combination thereof, or other touch screen or television with one or more processors embedded therein or coupled thereto, or any other sort of computer-related electronic device having network connectivity.

Electronic system 600 may include various types of computer readable media and interfaces for various other types of computer readable media. In the depicted example, electronic system 600 includes a bus 608, processing unit(s) 612, a system memory 604, a read-only memory (ROM) 610, a permanent storage device 602, an input device interface 614, an output device interface 606, and one or more network interfaces 616. In some implementations, electronic system 600 may include or be integrated with other computing devices or circuitry for operation of the various components and processes previously described.

Bus 608 collectively represents all system, peripheral, and chipset buses that communicatively connect the numerous internal devices of electronic system 600. For instance, bus 608 communicatively connects processing unit(s) 612 with ROM 610, system memory 604, and permanent storage device 602.

From these various memory units, processing unit(s) 612 retrieves instructions to execute and data to process in order to execute the processes of the subject disclosure. The processing unit(s) can be a single processor or a multi-core processor in different implementations.

ROM 610 stores static data and instructions that are needed by processing unit(s) 612 and other modules of the electronic system. Permanent storage device 602, on the other hand, is a read-and-write memory device. This device is a non-volatile memory unit that stores instructions and data even when electronic system 600 is off. Some implementations of the subject disclosure use a mass-storage device (such as a magnetic or optical disk and its corresponding disk drive) as permanent storage device 602.

Other implementations use a removable storage device (such as a floppy disk, flash drive, and its corresponding disk drive) as permanent storage device 602. Like permanent storage device 602, system memory 604 is a read-and-write memory device. However, unlike storage device 602, system memory 604 is a volatile read-and-write memory, such a random access memory. System memory 604 stores some of the instructions and data that the processor needs at runtime. In some implementations, the processes of the subject disclosure are stored in system memory 604, permanent storage device 602, and/or ROM 610. From these various memory units, processing unit(s) 612 retrieves instructions to execute and data to process in order to execute the processes of some implementations.

Bus 608 also connects to input and output device interfaces 614 and 606. Input device interface 614 enables the user to communicate information and select commands to the electronic system. Input devices used with input device interface 614 include, e.g., alphanumeric keyboards and pointing devices (also called "cursor control devices"). Output device interfaces 606 enables, e.g., the display of images generated by the electronic system 600. Output devices used with output device interface 606 include, e.g., printers and display devices, such as cathode ray tubes (CRT) or liquid crystal displays (LCD). Some implementations include devices such as a touchscreen that functions as both input and output devices.

Also, as shown in FIG. 6, bus 608 also couples electronic system 600 to a network (not shown) through network interfaces 616. Network interfaces 616 may include, e.g., a wireless access point (e.g., Bluetooth or WiFi) or radio circuitry for connecting to a wireless access point. Network interfaces 616 may also include hardware (e.g., Ethernet hardware) for connecting the computer to a part of a network of computers such as a local area network ("LAN"), a wide area network ("WAN"), wireless LAN, or an Intranet, or a network of networks, such as the Internet. Any or all components of electronic system 600 can be used in conjunction with the subject disclosure.

These functions described above can be implemented in computer software, firmware or hardware. The techniques can be implemented using one or more computer program products. Programmable processors and computers can be included in or packaged as mobile devices. The processes and logic flows can be performed by one or more programmable processors and by one or more programmable logic circuitry. General and special purpose computing devices and storage devices can be interconnected through communication networks.

Some implementations include specifically configured electronic components, such as microprocessors, storage and memory that store computer program instructions in a machine-readable or computer-readable medium (also referred to as computer-readable storage media, machine-readable media, or machine-readable storage media). Some examples of such computer-readable media include RAM, ROM, read-only compact discs (CD-ROM), recordable compact discs (CD-R), rewritable compact discs (CD-RW), read-only digital versatile discs (e.g., DVD-ROM, dual-layer DVD-ROM), a variety of recordable/rewritable DVDs (e.g., DVD-RAM, DVD-RW, DVD+RW, etc.), flash memory (e.g., SD cards, mini-SD cards, micro-SD cards, etc.), magnetic and/or solid state hard drives, read-only and recordable Blu-Ray^{®} discs, ultra-density optical discs, any other optical or magnetic media, and floppy disks. The computer-readable media can store a computer program that is executable by at least one processing unit and includes sets of instructions for performing various operations. Examples of computer programs or computer code include machine code, such as is produced by a compiler, and files including higher-level code that are executed by a computer, an electronic component, or a microprocessor using an interpreter.

While the above discussion primarily refers to microprocessor or multi-core processors that execute software, some implementations are performed by one or more integrated circuits, such as application specific integrated circuits (ASICs) or field programmable gate arrays (FPGAs). In some implementations, such integrated circuits execute instructions that are stored on the circuit itself.

As used in this specification and any claims of this application, the terms "computer", "server", "processor", and "memory" all refer to electronic or other technological devices. These terms exclude people or groups of people. For the purposes of the specification, the terms display or displaying means displaying on an electronic device. As used in this specification and any claims of this application, the terms "computer readable medium" and "computer readable media" are entirely restricted to tangible, physical objects that store information in a form that is readable by a computer. These terms exclude any wireless signals, wired download signals, and any other ephemeral signals.

Although described in some implementations as a light array, additional or alternative devices can be used to provide human perceivable infusion feedback such as an amplifier to provide auditory feedback or a haptic motor to provide tactile feedback.

Implementations of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), an internetwork (e.g., the Internet), and peer-to-peer networks (e.g., ad hoc peer-to-peer networks).

The computing system can include clients and servers. A client and server are generally remote from each other and may interact through a communication network. The relationship of client and server arises by virtue of computer programs running on the respective computers and having a client-server relationship to each other. In some implementations, a server transmits data (e.g., an HTML page) to a client device (e.g., for purposes of displaying data to and receiving user input from a user interacting with the client device). Data generated at the client device (e.g., a result of the user interaction) can be received from the client device at the server.

Those of skill in the art would appreciate that the various illustrative blocks, modules, elements, components, methods, and algorithms described herein may be implemented as electronic hardware, computer software, or combinations of both. To illustrate this interchangeability of hardware and software, various illustrative blocks, modules, elements, components, methods, and algorithms have been described above generally in terms of their functionality. Whether such functionality is implemented as hardware or software depends upon the particular application and design constraints imposed on the overall system. The described functionality may be implemented in varying ways for each particular application. Various components and blocks may be arranged differently (e.g., arranged in a different order, or partitioned in a different way) all without departing from the scope of the subject technology.

### Further Consideration:

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

The previous description is provided to enable any person skilled in the art to practice the various aspects described herein. The previous description provides various examples of the subject technology, and the subject technology is not limited to these examples. Various modifications to these aspects will be readily apparent to those skilled in the art, and the generic principles defined herein may be applied to other aspects. Thus, the claims are not intended to be limited to the aspects shown herein, but is to be accorded the full scope consistent with the language claims, wherein reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention described herein.

The term website, as used herein, may include any aspect of a website, including one or more web pages, one or more servers used to host or store web related content, etc. Accordingly, the term website may be used interchangeably with the terms web page and server. The predicate words "configured to", "operable to", and "programmed to" do not imply any particular tangible or intangible modification of a subject, but, rather, are intended to be used interchangeably. For example, a processor configured to monitor and control an operation or a component may also mean the processor being programmed to monitor and control the operation or the processor being operable to monitor and control the operation. Likewise, a processor configured to execute code can be construed as a processor programmed to execute code or operable to execute code.

The term automatic, as used herein, may include performance by a computer or machine without user intervention; for example, by instructions responsive to a predicate action by the computer or machine or other initiation mechanism. The word "example" is used herein to mean "serving as an example or illustration." Any aspect or design described herein as "example" is not necessarily to be construed as preferred or advantageous over other aspects or designs.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all implementations, or one or more implementations. An embodiment may provide one or more examples. A phrase such as an "embodiment" may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such as a "configuration" may refer to one or more configurations and vice versa.

## Claims

1. An infusion set comprising:
an fluid-carrying tubing;
a light array coupled to the fluid-carrying tubing at a plurality of fastening points along a length of the fluid-carrying tubing;
a pump interface; and
a controller, wherein the controller is configured to:
receive a pumping parameter from the pump interface, the pumping parameter being generated by an infusion pump providing a fluid to a patient through the fluid-carrying tubing and representative of a property of the fluid that is measured by a sensor of the infusion pump; the infusion set being **characterized in that** the controller is further configured to cause, responsive to receiving the pumping parameter, the light array to display, along the length of the fluid-carrying tubing, a light pattern representative of the pumping parameter.

2. The infusion set of claim 1, wherein the controller is configured to:
store a history of pumping parameters, including the received pumping parameters, in a memory device within the infusion set, wherein the light pattern is based on the stored history of pumping parameters.

3. The infusion set of claim 1 or claim 2, wherein the received pumping parameter is indicative of a fault condition associated with providing the fluid to the patient through the fluid-carrying tube.

4. The infusion set of any one of claims 1 through 3, wherein causing the light array to display the light pattern comprises causing the light array to flash.

5. The infusion set of any one of claims 1 through 3, wherein causing the light array to display the light pattern comprises modulating a subset of the light array in a sequence that designates a location associated with the fault condition.

6. The infusion set of claim 5, wherein the fault condition is upstream of the infusion pump or downstream of the infusion pump, and wherein when the fault condition is upstream of the infusion pump a plurality of lights of the light array between the infusion pump and a fluid source are modulated according to the sequence, and wherein when the fault condition is downstream of the infusion pump a plurality of lights of the light array between the infusion pump and the patient are modulated according to the sequence.

7. The infusion set of any one of claims 1 through 6, wherein the pump interface comprises a wireless communication device and is configured to communicate with the infusion pump via a wireless connection, the pumping parameter being wirelessly received from the infusion pump.

8. The infusion set of any one of claims 1 through 7, wherein light array is embedded in a light string and the plurality of fastening points comprise clips integrated with the light string and which clip onto the fluid-carrying tubing at periodic intervals along the fluid-carrying tubing.

9. The infusion set of any one of claims 1 through 8, wherein the light array comprises a plurality of lights embedded within a casing the runs along a length of the fluid-carrying tubing.

10. A method comprising:
providing a light array configured to couple to a fluid-carrying tubing at a plurality of fastening points along a length of the fluid-carrying tubing;
measuring a property of the fluid in the fluid-carrying tubing;
receiving, by a controller operably coupled to the light array, a pumping parameter from a pump interface, the pumping parameter representative of the measured property and being generated by an infusion pump providing a fluid to a patient through the fluid-carrying tubing; and
causing, responsive to receiving the pumping parameter, the light array to display, along the length of the fluid-carrying tubing, a light pattern representative of the pumping parameter.

11. The method of claim 10 or claim 11, further comprising:
storing a history of pumping parameters, including the received pumping parameters, in a memory device, wherein the light pattern is based on the stored history of pumping parameters.

12. The method of any one of claims 10 through 11, wherein the received pumping parameter is indicative of a fault condition associated with providing the fluid to the patient through the fluid-carrying tube.

13. The method of any one of claims 10 through 12, wherein causing the light array to display the light pattern comprises causing the light array to flash.

14. The method of any one of claims 10 through 13, wherein causing the light array to display the light pattern comprises modulating a subset of the light array in a sequence that designates a location associated with the fault condition.

15. The method of claim 14, wherein the fault condition is upstream of the infusion pump or downstream of the infusion pump, and wherein when the fault condition is upstream of the infusion pump a plurality of lights in the light array between the infusion pump and a fluid source are modulated according to the sequence, and wherein when the fault condition is downstream of the infusion pump a plurality of lights in the light array between the infusion pump and the patient are modulated according to the sequence.

16. The method of any one of claims 10 through 15, further comprising:
wirelessly receiving, via a wireless interface, the pumping parameter from the infusion pump.

17. The method of any one of claims 10 through 16, wherein light array is embedded in a light string and the plurality of fastening points comprise clips integrated with the light string and which clip onto the fluid-carrying tubing at periodic intervals along the fluid-carrying tubing.

18. The method of any one of claims 10 through 17, wherein the light array comprises a plurality of lights embedded within a casing the runs along a length of the fluid-carrying tubing.

19. The method of any one of claims 10 through 18, further comprising:
determining that the fluid-carrying tubing has been in use for longer than a predetermined period of time; and
modulating the lights to display a pattern representative of the predetermined period of time being exceeded.

20. An infusion safety device comprising:
a light array;
a plurality of fasteners to physically couple the light array to a length of a fluid-carrying tubing;
a pump interface; and
a controller, wherein the controller is configured to:
receive a pumping parameter from the pump interface, the pumping parameter being generated by an infusion pump providing a fluid to a patient through the fluid-carrying tubing and representative of a property of the fluid that is measured by a sensor of the infusion pump; the infusion safety device being **characterized in that** the controller is further configured to cause, responsive to receiving the pumping parameter, the light array to display, along the length of the fluid-carrying tubing, a light pattern representative of the pumping parameter.

## Patentansprüche

1. Ein Infusionsset, umfassend:
einen flüssigkeitsführenden Schlauch;
eine Lichtanordnung, die mit dem flüssigkeitsführenden Schlauch an einer Vielzahl von Befestigungspunkten entlang einer Länge des flüssigkeitsführenden Schlauchs verbunden ist;
eine Pumpenschnittstelle; und
eine Steuereinheit, wobei die Steuereinheit konfiguriert ist, um:
einen Pumpparameter von der Pumpenschnittstelle zu empfangen, wobei der Pumpparameter von einer Infusionspumpe erzeugt wird, die einem Patienten durch den flüssigkeitsführenden Schlauch eine Flüssigkeit zuführt, und für eine Eigenschaft der Flüssigkeit repräsentativ ist, die von einem Sensor der Infusionspumpe gemessen wird; wobei das Infusionsset **dadurch gekennzeichnet ist, dass** die Steuereinheit ferner konfiguriert ist, um
als Reaktion auf den Empfang des Pumpparameters die Lichtanordnung zu veranlassen, entlang der Länge des flüssigkeitsführenden Schlauchs ein für den Pumpparameter repräsentatives Lichtmuster anzuzeigen.

2. Infusionsset nach Anspruch 1, wobei die Steuereinheit so konfiguriert ist, um:
einen Verlauf von Pumpparametern, einschließlich der empfangenen Pumpparameter, in einer Speichervorrichtung innerhalb des Infusionssets zu speichern, wobei das Lichtmuster auf dem gespeicherten Verlauf von Pumpparametern basiert.

3. Infusionsset nach Anspruch 1 oder Anspruch 2, wobei der empfangene Pumpparameter einen Fehlerzustand anzeigt, der mit der Bereitstellung der Flüssigkeit an den Patienten durch den flüssigkeitsführenden Schlauch verbunden ist.

4. Infusionsset nach einem der Ansprüche 1 bis 3, wobei das Veranlassen der Lichtanordnung zur Anzeige des Lichtmusters das Veranlassen der Lichtanordnung zum Blinken umfasst.

5. Infusionsset nach einem der Ansprüche 1 bis 3, wobei das Veranlassen der Lichtanordnung zur Anzeige des Lichtmusters das Modulieren einer Untergruppe der Lichtanordnung in einer Sequenz umfasst, die eine mit dem Fehlerzustand verbundene Stelle bezeichnet.

6. Infusionsset nach Anspruch 5, wobei der Fehlerzustand stromaufwärts von der Infusionspumpe oder stromabwärts von der Infusionspumpe liegt, und wobei, wenn der Fehlerzustand stromaufwärts von der Infusionspumpe liegt, eine Vielzahl von Lichtern der Lichtanordnung zwischen der Infusionspumpe und einer Flüssigkeitsquelle gemäß der Sequenz moduliert wird, und wobei, wenn der Fehlerzustand stromabwärts von der Infusionspumpe liegt, eine Vielzahl von Lichtern der Lichtanordnung zwischen der Infusionspumpe und dem Patienten gemäß der Sequenz moduliert wird.

7. Infusionsset nach einem der Ansprüche 1 bis 6, wobei die Pumpenschnittstelle eine drahtlose Kommunikationsvorrichtung umfasst und so konfiguriert ist, dass sie mit der Infusionspumpe über eine drahtlose Verbindung kommuniziert, wobei der Pumpparameter drahtlos von der Infusionspumpe empfangen wird.

8. Infusionsset nach einem der Ansprüche 1 bis 7, wobei die Lichtanordnung in eine Lichterkette eingebettet ist und die mehreren Befestigungspunkte Clips umfassen, die in die Lichterkette integriert sind und in periodischen Abständen entlang des flüssigkeitsführenden Schlauchs an diesem befestigt werden.

9. Infusionsset nach einem der Ansprüche 1 bis 8, wobei die Lichtanordnung eine Vielzahl von Lichtern umfasst, die in ein Gehäuse eingebettet sind, das entlang der Länge des flüssigkeitsführenden Schlauchs verläuft.

10. Ein Verfahren, das Folgendes umfasst:
Bereitstellen einer Lichtanordnung, die so konfiguriert ist, dass sie an einer Vielzahl von Befestigungspunkten entlang einer Länge eines flüssigkeitsführenden Schlauchs mit dem flüssigkeitsführenden Schlauch verbunden werden kann;
Messen einer Eigenschaft der Flüssigkeit in dem flüssigkeitsführenden Schlauch;
Empfangen eines Pumpparameters von einer Pumpenschnittstelle durch eine Steuereinheit, die funktionsfähig mit der Lichtanordnung gekoppelt ist, wobei der Pumpparameter die gemessene Eigenschaft darstellt und von einer Infusionspumpe erzeugt wird, die einem Patienten durch den flüssigkeitsführenden Schlauch eine Flüssigkeit zuführt; und
Bewirken, als Reaktion auf den Empfang des Pumpparameters, dass die Lichtanordnung entlang der Länge des flüssigkeitsführenden Schlauchs ein Lichtmuster anzeigt, das den Pumpparameter darstellt.

11. Verfahren nach Anspruch 10 oder 11, ferner umfassend:
Speichern eines Verlaufs von Pumpparametern, einschließlich der empfangenen Pumpparameter, in einer Speichervorrichtung, wobei das Lichtmuster auf dem gespeicherten Verlauf von Pumpparametern basiert.

12. Verfahren nach einem der Ansprüche 10 bis 11, wobei der empfangene Pumpparameter einen Fehlerzustand anzeigt, der mit der Bereitstellung der Flüssigkeit an den Patienten durch den flüssigkeitsführenden Schlauch verbunden ist.

13. Verfahren nach einem der Ansprüche 10 bis 12, wobei das Veranlassen der Lichtanordnung zur Anzeige des Lichtmusters das Veranlassen der Lichtanordnung zum Blinken umfasst.

14. Verfahren nach einem der Ansprüche 10 bis 13, wobei das Veranlassen der Lichtanordnung zur Anzeige des Lichtmusters das Modulieren einer Untergruppe der Lichtanordnung in einer Sequenz umfasst, die eine mit dem Fehlerzustand verbundene Stelle bezeichnet.

15. Verfahren nach Anspruch 14, wobei der Fehlerzustand stromaufwärts von der Infusionspumpe oder stromabwärts von der Infusionspumpe liegt, und wobei, wenn der Fehlerzustand stromaufwärts von der Infusionspumpe liegt, eine Vielzahl von Lichtern in der Lichtanordnung zwischen der Infusionspumpe und einer Flüssigkeitsquelle gemäß der Sequenz moduliert wird, und wobei, wenn der Fehlerzustand stromabwärts von der Infusionspumpe liegt, eine Vielzahl von Lichtern in der Lichtanordnung zwischen der Infusionspumpe und dem Patienten gemäß der Sequenz moduliert wird.

16. Verfahren nach einem der Ansprüche 10 bis 15, ferner umfassend:
drahtloses Empfangen des Pumpparameters von der Infusionspumpe über eine drahtlose Schnittstelle.

17. Verfahren nach einem der Ansprüche 10 bis 16, wobei die Lichtanordnung in eine Lichterkette eingebettet ist und die mehreren Befestigungspunkte Clips umfassen, die in die Lichterkette integriert sind und in periodischen Abständen entlang des flüssigkeitsführenden Schlauchs an diesem befestigt sind.

18. Verfahren nach einem der Ansprüche 10 bis 17, wobei die Lichtanordnung eine Vielzahl von Lichtern umfasst, die in ein Gehäuse eingebettet sind, das entlang einer Länge des flüssigkeitsführenden Schlauchs verläuft.

19. Verfahren nach einem der Ansprüche 10 bis 18, ferner umfassend:
Feststellen, dass der flüssigkeitsführende Schlauch länger als eine vorbestimmte Zeitspanne in Gebrauch war; und
Modulieren der Lichter, um ein Muster anzuzeigen, das die Überschreitung der vorbestimmten Zeitspanne darstellt.

20. Infusionssicherheitsvorrichtung, umfassend:
eine Lichtanordnung;
eine Vielzahl von Befestigungselementen zum physischen Koppeln der Lichtanordnung mit einer Länge eines flüssigkeitsführenden Schlauchs;
eine Pumpenschnittstelle; und
eine Steuereinheit, wobei die Steuereinheit so konfiguriert ist, um:
einen Pumpparameter von der Pumpenschnittstelle zu empfangen, wobei der Pumpparameter von einer Infusionspumpe erzeugt wird, die einem Patienten eine Flüssigkeit durch den flüssigkeitsführenden Schlauch zuführt, und eine Eigenschaft der Flüssigkeit darstellt, die von einem Sensor der Infusionspumpe gemessen wird; wobei die Infusionssicherheitsvorrichtung **dadurch gekennzeichnet ist, dass** die Steuereinheit ferner konfiguriert ist, um
als Reaktion auf den Empfang des Pumpparameters die Lichtanordnung zu veranlassen, entlang der Länge des flüssigkeitsführenden Schlauchs ein für den Pumpparameter repräsentatives Lichtmuster anzuzeigen.

## Revendications

1. Kit de perfusion comprenant :
une tubulure de transport de fluide
un réseau lumineux couplé à la tubulure de transport de fluide en plusieurs points de fixation sur la longueur de la tubulure de transport de fluide ;
une interface de pompe ; et
un contrôleur, dans lequel le contrôleur est configuré pour :
recevoir un paramètre de pompage de l'interface de pompe, le paramètre de pompage étant généré par une pompe à perfusion fournissant un fluide à un patient à travers la tubulure de transport de fluide et représentant une propriété du fluide qui est mesurée par un capteur de la pompe à perfusion ; le kit de perfusion étant **caractérisé en ce que** le contrôleur est en outre configuré pour
causé, suite à la réception du paramètre de pompage, le réseau lumineux affiche, sur toute la longueur de la tubulure de transport de fluide, un motif lumineux représentatif du paramètre de pompage.

2. Le kit de perfusion de la revendication 1, dans lequel le contrôleur est configuré pour :
stocker un historique des paramètres de pompage, y compris les paramètres de pompage reçus, dans un dispositif de mémoire à l'intérieur du dispositif de perfusion, dans lequel le motif lumineux est basé sur l'historique stocké des paramètres de pompage.

3. Le kit de perfusion de la revendication 1 ou de la revendication 2, dans lequel le paramètre de pompage reçu est indicatif d'une condition de défaut associée à l'administration du fluide au patient par la tubulure de transport de fluide.

4. Le kit de perfusion de l'une des revendications 1 à 3, dans lequel l'affichage du motif lumineux par le réseau lumineux consiste à faire clignoter le réseau lumineux.

5. Le kit de perfusion de l'une des revendications 1 à 3, dans lequel le fait d'amener le réseau lumineux à afficher le motif lumineux consiste à moduler un sous-ensemble de le réseau lumineux dans une séquence qui désigne un emplacement associé à la condition d'erreur.

6. Le kit de perfusion de la revendication 5, dans lequel la condition d'erreur est en amont de la pompe à perfusion ou en aval de la pompe à perfusion, et dans lequel lorsque la condition d'erreur est en amont de la pompe à perfusion, une pluralité de lumières du réseau lumineux entre la pompe à perfusion et une source de fluide sont modulées selon la séquence, et dans lequel lorsque la condition d'erreur est en aval de la pompe à perfusion, une pluralité de lumières du réseau lumineux entre la pompe à perfusion et le patient sont modulées selon la séquence.

7. Le kit de perfusion de l'une des revendications 1 à 6, dans lequel l'interface de pompe comprend un dispositif de communication sans fil et est configuré pour communiquer avec la pompe à perfusion via une connexion sans fil, le paramètre de pompage étant reçu sans fil de la pompe à perfusion.

8. Le kit de perfusion de l'une des revendications 1 à 7, dans lequel le réseau lumineux est intégré dans une guirlande lumineuse et la pluralité de points de fixation comprend des clips intégrés à la guirlande lumineuse et qui se clipsent sur la tubulure de transport de fluide à intervalles périodiques le long de la tubulure de transport de fluide.

9. Le kit de perfusion de l'une des revendications 1 à 8, dans lequel le réseau lumineux comprend une pluralité de lumières intégrées dans un boîtier qui s'étend sur la longueur de la tubulure de transport de fluide.

10. Une méthode comprenant :
fournir un réseau lumineux configuré pour se coupler à une tubulure de transport de fluide à une pluralité de points de fixation sur la longueur de la tubulure de transport de fluide ;
mesurer d'une propriété du fluide dans la tubulure de transport de fluide ;
recevoir, par un contrôleur couplé de manière opérationnelle à le réseau lumineux, un paramètre de pompage provenant d'une interface de pompe, le paramètre de pompage représentant la propriété mesurée et étant généré par une pompe à perfusion fournissant un fluide à un patient par l'intermédiaire de la tubulure de transport de fluide ; et
causer que, suite à la réception du paramètre de pompage, le réseau lumineux affiche, sur la longueur de la tubulure de transport de fluide, un motif lumineux représentatif du paramètre de pompage.

11. La méthode de la revendication 10 ou de la revendication 11, comprenant en outre :
stocker un historique des paramètres de pompage, y compris les paramètres de pompage reçus, dans un dispositif de mémoire, dans lequel le motif lumineux est basé sur l'historique stocké des paramètres de pompage.

12. La méthode de l'une des revendications 10 à 11, dans laquelle le paramètre de pompage reçu est indicatif d'une condition de défaut associée à la fourniture du fluide au patient par la tubulure de transport de fluide.

13. La méthode de l'une des revendications 10 à 12, dans laquelle causer l'affichage du motif lumineux par le réseau lumineux consiste à faire clignoter le réseau lumineux.

14. La méthode de l'une des revendications 10 à 13, dans laquelle causer d'amener le réseau lumineux à afficher le motif lumineux consiste à moduler un sous-ensemble de le réseau lumineux dans une séquence qui désigne un emplacement associé à la condition de défaut.

15. La méthode de la revendication 14, dans laquelle la condition de défaut est en amont de la pompe à perfusion ou en aval de la pompe à perfusion, et dans laquelle, lorsque la condition de défaut est en amont de la pompe à perfusion, une pluralité de lumières dans le réseau lumineux entre la pompe à perfusion et une source de fluide sont modulées selon la séquence, et dans laquelle, lorsque la condition de défaut est en aval de la pompe à perfusion, une pluralité de lumières dans le réseau lumineux entre la pompe à perfusion et le patient sont modulées selon la séquence.

16. La méthode de l'une des revendications 10 à 15, comprenant en outre :
la réception sans fil, via une interface sans fil, du paramètre de pompage de la pompe à perfusion.

17. La méthode de l'une des revendications 10 à 16, dans laquelle le réseau lumineux est intégré dans une guirlande lumineuse et la pluralité de points de fixation comprend des clips intégrés à la guirlande lumineuse et qui se fixent sur la tubulure de transport de fluide à intervalles périodiques le long de la tubulure de transport de fluide.

18. La méthode de l'une des revendications 10 à 17, dans laquelle le réseau lumineux comprend une pluralité de lumières intégrées dans un boîtier qui s'étend sur la longueur de la tubulure de transport de fluide.

19. La méthode de l'une des revendications 10 à 18, comprenant en outre :
déterminer que la tubulure de transport de fluide a été utilisé pendant plus longtemps qu'une période de temps prédéterminée ; et
moduler les lumières pour afficher un motif représentatif du dépassement de la période prédéterminée.

20. Dispositif de sécurité pour perfusion comprenant :
un réseau lumineux ;
une pluralité d'attaches pour coupler physiquement le réseau lumineux à une longueur de tubulure de transport de fluide ;
une interface de pompe ; et
un contrôleur, dans lequel le contrôleur est configuré pour :
recevoir un paramètre de pompage de l'interface de pompe, le paramètre de pompage étant généré par une pompe à perfusion fournissant un fluide à un patient à travers la tubulure de transport de fluide et représentant une propriété du fluide mesurée par un capteur de la pompe à perfusion ; le dispositif de sécurité pour perfusion est **caractérisé en ce que** le contrôleur est en outre configuré pour
causer que, suite à la réception du paramètre de pompage, le réseau lumineux affiche, sur la longueur de la tubulure de transport de fluide, un motif lumineux représentatif du paramètre de pompage.
